# FASCICULE DE BREVET EUROPEEN

(11) **EP 0 588 707 B1**
(45) Date de publication et mention de la délivrance du brevet: **24.07.1996**
(21) Numéro de dépôt: 93402227.8
(22) Date de dépôt: 14.09.1993
(51) Int. Cl.: A23K 1/18, A23K 1/16, A61K 9/56, A61K 47/36, A61K 47/44

(54) **Compositions nutritives ou médicamenteuses pour l'administration aux ruminants à base de chitosane**
Nährstoff- und Arzneimittelzubereitungen auf der Basis von Chitosan für die Verabreichung an Wiederkäuer
Chitosan-based nutrient and drug compositions for administration to ruminants

(30) Priorité: 18.09.1992 FR 9211129
(43) Date de publication de la demande: 23.03.1994
(73) Titulaire: RHONE-POULENC NUTRITION ANIMALE, 92160 Antony (FR)
(72) Inventeur: Prud'Homme, Christian, F-69006 Lyon (FR); Rostaing, Jean-François, F-38200 Chuzelles (FR)
(74) Mandataire: Le Pennec, Magali

(56) Documents cités:
- EP-A- 0 478 783
- WO-A-88/00237
- FR-A- 2 524 269
- GB-A- 2 160 096
- DATABASE WPI Week 8325, Derwent Publications Ltd., London, GB; AN 83-61040K & SU-A-950 261 (FAR E FISH IND TECH) 15 Aoüt 1982
- DATABASE WPI Week 9244, Derwent Publications Ltd., London, GB; AN 92-361898 & JP-A-4 264 023 (TEIKOKU SEIYAKU KK) 18 Septembre 1992
- DATABASE WPI Week 8051, Derwent Publications Ltd., London, GB; AN 80-91053C & JP-A-55 141 171 (KURARAY KK) 5 Novembre 1980
- CHEMICAL ABSTRACTS, vol. 111, no. 9, 28 Aoüt 1989, Columbus, Ohio, US; abstract no. 78544k, page 802 ;colonne 1 ;

## Description

La présente invention concerne de nouvelles compositions pour l'alimentation des ruminants, contenant une ou plusieurs substances biologiquement actives, qui sont stables dans un milieu aqueux dont le pH est égal ou supérieur à 5,5 qui est le milieu du rumen et qui permettent la libération de la ou des substances actives dans le tractus digestif dans un compartiment post rumen.

L'invention concerne plus particulièrement de nouvelles compositions pour l'alimentation des ruminants, contenant une ou plusieurs substances nutritives ou une ou plusieurs substances thérapeutiques, qui en l'absence de la composition protectrice seraient détruites dans la panse des ruminants en raison de la présence des microorganismes résidant dans la panse qui favorisent la digestion des matières amylacées. Le temps de séjour dans la panse des principes actifs est souvent d'une durée moyenne de 15 à 20 heures. Pendant ce laps de temps relativement long, les principes actifs fragiles ont largement le temps d'être détruits. Afin de protéger ces substances et de pouvoir ensuite les libérer dans le tractus digestif, en un lieu moins agressif pour elles, de nombreuses solutions ont déjà été proposées.

Parmi les composés permettant une protection dans le milieu du rumen et une liberation dans la caillette ou les intestins on peut citer deux grands types de composés. Certains de ces composés permettent une libération qui est provoquée uniquement par un phénomène chimique, dû à la différence de nature du pH entre le rumen et la caillette; on peut citer dans cette catégorie d'abord les polymères pH sensibles tels que les copolymères du styrène et de la vinylpyridine. De nombreux brevets tels que par exemple les brevets US 4 877 621 ou US 4 832 967 décrivent des compositions contenant de tels composés. Ces compositions permettent une excellente protection des substances nutritives ou médicamenteuses mais présentent un seul inconvénient, elles contiennent un produit chimique de synthèse qui d'un point de vue alimentaire et écologique n'est pas toujours très apprécié tant du point de vue des autorités administratives d'autorisation de mise sur le marché que des consommateurs.

Dans cette première catégorie de compositions où la libération est provoquée uniquement par un phénomène chimique, on peut citer aussi, les compositions à base de chitosane et d'acides carboxyliques. Ces compositions sont par exemple décrites dans le brevet français publié sous le numéro 2 524 269. Ces compositions contiennent une quantité de principes actifs toujours inférieure à 60 % en poids et plus préférentiellement de l'ordre de 30 % pour pouvoir permettre une protection au niveau du rumen de l'ordre de 80 à 90 % sur 24 heures et contiennent, en outre, une ou plusieurs charges minérales pH sensibles, en quantité notable (10 à 20 % en poids).

Une deuxième catégorie de brevets décrivent des compositions utilisant les propriétés d'hydrolyse enzymatique de certains composés naturels tels que la zéine. Parmi ces brevets on peut citer le brevet US 4 983 403 ou la demande de brevet européen EP 0 406 041. Ces compositions permettent comme les précédentes une excellente protection du principe actif au niveau du rumen ainsi qu'une bonne libération post rumen, mais la zéine présente un inconvénient majeur, elle n'est soluble que dans les solvants organiques ce qui oblige l'industrie à la pulvériser sur les principes actifs par une technique de pulvérisation de la zéine mise en solution dans un solvant et exige donc en conséquence une récupération des dits solvants fort couteuse.

L'objectif que cherche à atteindre l'ensemble de ces compositions pour l'alimentation des ruminants est de pouvoir conserver environ 80% du ou des principes actifs dans le rumen sans dégradation pendant 6 heures minimum et encore plus avantageusement pendant 24 heures minimum et de permettre une libération d'au moins 50% et de préférence d'au moins 80% du ou des mêmes principes actifs en moins de 6 heures dans la caillette et/ou l'intestin. Le but recherché, en plus des objectifs précédemment énoncés, dans le cadre de la présente invention, est de disposer d'une composition contenant au moins 60 % en poids de principe actif et contenant la plus grande quantité possible de composés protecteurs d'origine naturelle et reconnus comme ayant une qualité alimentaire pour les animaux.

Ce but a été atteint par l'utilisation pour l'alimentation animale d'une composition nutritive ou médicamenteuse à base d'une ou plusieurs substances biologiquement actives enrobées par une composition d'enrobage contenant en poids :
- 1 à 10 % de sels de chitosane exprimé en équivalent chitosane
- 99 à 90 % d'un corps gras ou d'un mélange de corps gras dont le point de fusion est supérieur à 45°C.

Selon une préférence, la composition d'enrobage contient 1 à 5 % de sels de chitosane. Dans toute la description le taux de sels de chitosane est exprimé en équivalents chitosane.

Cette composition d'enrobage contient encore plus préférentiellement en poids :
- 3 à 5 % de sels de chitosane
- 45 à 97 % de corps gras ayant un point de fusion supérieur à 50°C
- 0 à 50 % d'un corps gras ou d'un mélange de corps gras ayant un point de fusion inférieur à 25 °C.

Dans ces compositions d'enrobage les sels de chitosane peuvent être choisis parmi le chlorure, l'acétate, l'adipate, le citrate, le formiate, le glutamate, le lactate, le malonate, l'oxalate, le propionate, la pyruvate, le succinate, le tartrate, ainsi que tous les sels qui permettent de solubiliser le chitosane dans l'eau. Parmi tous ces sels on préfère utiliser l'acétate de chitosane.

Parmi les corps gras présentant un point de fusion supérieur à 50°C on peut citer, à titre d'exemple, les acides gras, tels que par exemple l'acide stéarique, l'acide béhénique, laurique et myristique ou leur mélange tel que la stéarine, les esters d'acides gras et par exemple de glycérol, les alcools gras dont la chaine grasse contient 12 à 22 atomes de carbone, les paraffines, les huiles végétales ou animales hydrogénées, les cires comme notamment les cires de Carnauba ou d'abeille. Ces corps gras peuvent s'utiliser à l'état pur, mais ils seront encore plus préférentiellement utilisés en un mélange tel que, par exemple, la stéarine.

Les esters d'acide gras et de glycérol peuvent être choisis parmi les monoesters, les diesters ou les triester de glycérol, ces esters sont souvent commercialisés en mélange. Ainsi le mélange de monoester et de triester de béhénate de glycérol est comercialisé par exemple par la société Gatefossé sous la dénomination commerciale de Compritol, le mélange de monopalmitate et de monostéarate de glycérol est commercialisé sous la dénomination commerciale de Géléol.

Parmi les corps gras ayant un point de fusion inférieur à 25°C on peut citer, encore à titre d'exemple, les acides gras insaturés, ayant 12 à 22 atomes de carbone, pur tel que l'acide oléique ou en mélange comme par exemple les huiles alimentaires (colza, tournesol, coprah, arachide, maïs, olive).

Parmi les compositions d'enrobage tout à fait préférentielles, on peut citer les compositions suivantes :
- 3 à 5 % de sels de chitosane
- 55 à 92 % d'un corps gras dont le point de fusion est supérieur à 50°C
- 5 à 40 % d'un ou plusieurs corps gras dont le point de fusion est inférieur à 25°C.

Le corps gras ayant un point de fusion supérieur à 50°C est de préférence l'acide stéarique, le corps gras ayant un point de fusion inférieur à 25°C est de préférence l'acide oléique.

La composition la plus préférentielle pour enrober la méthionine contient en poids :
- 3 % d'acétate de chitosane
- 90 % d'acide stéarique
- 7 % d'acide oléique.

Les compositions nutritives ou médicamenteuses préférées selon l'invention contiennent 60 à 90 % et de préférence 70 à 80 % en poids de principes actifs dégradables dans la panse des ruminants et contiennent 4 à 25 %, de préférence 10 à 25 % en poids de composition d'enrobage selon l'invention.

Le complément à 100 % de la composition nutritive ou médicamenteuse est constitué par l'agent liant qui est utilisé notamment conformément au brevet FR90 08280 pour former le granulé.

Les principes actifs alimentaires sont choisis notamment parmi les acides aminés essentiels supposés limitant tels que la méthionine, la lysine et/ou le tryptophane. Les principes actifs médicamenteux sont choisis notamment parmi les vitamines, les antibiotiques, les composés antiparasitaires ou les hormones. L'ensemble de ces principes actifs sont des principes biologiquement actifs.

La présente invention permet d'obtenir un taux de principes actifs dans la composition finale compris entre 60 et 90 % et plus préférentiellement compris entre 70 et 80%. La couche d'enrobage présente de préférence une épaisseur comprise entre 20 µm et 200 µm et plus préférentiellement compris entre 60 µm et 120 µm. Le granulé de principe actif a de préférence un diamètre compris entre 0,6 et 2,5 mm encore plus préférentiellement compris entre 0,8 et 2 mm.

Le procédé de préparation des compositions nutritives ou médicamenteuses selon l'invention permet d'éviter l'usage de tout solvant organique, il consiste à réaliser une émulsion aqueuse des constituants de la composition d'enrobage, puis à pulvériser cette émulsion sur les granulés de principes actifs. Selon une meilleure manière de mettre en oeuvre l'invention, on prépare une solution à environ 2 % en poids de sels de chitosane dans de l'eau, si l'on part d'un sel de chitosane à l'état solide, ou encore la solution aqueuse de chitosane peut être préparée de façon extemporanée, par dissolution du chitosane dans de l'eau contenant l'acide organique ou inorganique formant un sel soluble du chitosane.

A cette solution, que l'on peut éventuellement diluer, on ajoute, selon une meilleure façon de mettre en oeuvre l'invention, le corps gras ayant un point de fusion inférieur à 25°C puis à l'état fondu le corps gras ou le mélange de corps gras ayant un point de fusion supérieur à 50°C, on agite avec un appareil par exemple de type Polytron. On obtient une émulsion stable qui est facilement pulvérisée sur un lit de granulés à enrober par la technique du lit fluidisé de type par exemple Wurster telle que décrite dans les brevets US 2 799 241 et EP 0 188 953.

Les granulés obtenus après enrobage sont utilisés pour l'alimentation ou le traitement médicamenteux des ruminants.

L'invention sera plus complètement décrite à l'aide des exemples suivants qui ne doivent pas être considérés comme limitatifs de l'invention.

### EXEMPLES

### 1. Préparation d'une solution acétique de chitosane :

Dans un récipient de 3 litres en verre, on charge :
- 15 g de chitosane
- 580,4 g d'eau déminéralisée
- 4,62 g d'acide acétique à 100 %
Le mélange est agité à la température ambiante jusqu'à dissolution du chitosane. La solution est ensuite filtrée sur toile polyester à mailles de 60 µm afin d'éliminer les particules solides insolubles (0,41 g).

### 2. Préparation d'une émulsion de pelliculage selon l'exemple 3:

On prend la solution précédente que l'on dilue avec 600 g d'eau déminéralisée. On obtient ainsi une solution de chitosane à 1,2 %. On charge 209,7 g de cette solution dans un récipient de deux litres en verre. Après avoir ajouté à cette solution 0,425 g d'acide oléique purifié PROLABO, on chauffe le mélange jusqu'à 90-95°C avec un bain d'eau.

On coule ensuite en trois minutes environ, 76,5 g d'acide stéarique PRIFAC 2981 (UNICHEMA), mélangé à 5,525 g d'acide oléique purifié PROLABO ou d'un mélange tel qu'indiqué dans les tableaux 1 à 6, préalablement fondu dans une ampoule de coulée à double enveloppe chauffée à 110°C Durant la coulée, le mélange est dispersé à l'aide d'un appareil POLYTRON tournant à 15000 tours par minute. Le brassage avec le POLYTRON est maintenu encore pendant deux minutes après la fin de la coulée des lipides fondus.

On obtient une dispersion homogène et stable que l'on conserve à une température voisine de 90°C.

### 3. Enrobage de granulés de méthionine ou de granulés de méthionine chlorhydrate de lysine (exemples 3 et 18)

Dans un appareil de spray-coating UNIGLATT équipé d'un système WÜRSTER, on charge 500 g de granulés de méthionine ou de cogranulés méthionine/chlorhydrate de lysine préparés par extrusion sphéronisation fondu selon la demande de brevet FR 90 08280. Ces granulés ont des diamètres de particules compris entre 2 et 2,5 mm. Leur titre en méthionine est de 88,8 % pour les granulés de méthionine seule et d'environ 47 % en lysine, HCl et 13 % en méthionine pour les cogranulés.

L'émulsion précédente, toujours maintenue à 95°C, est ensuite pompée et pulvérisée dans l'appareil UNIGLATT sur le lit de particules fluidisées par un courant d'air chaud.

On applique les conditions de pelliculage suivantes :
- débit d'air de fluidisation : 130 m³/h
- température d'air de fluidisation (en sortie) : 40°C (48-50°C pour les cogranulés),
- pression d'air de pulvérisation : 1,5 bar
- débit d'émulsion d'enrobage : 10,4 g/mn (14,1 g/mn pour les c cogranulés)
- température d'air de pulvérisation : 90°C (80°C pour les cogranulés)
- durée de pulvérisation : 40 mn
Quantité totale d'émulsion pulvérisée : environ 414g pour la méthionine seule et d'environ 620g pour les cogranulés de lysine/méthionine.

On obtient ainsi 567,5 g de granulés pelliculés ayant un titre en méthionine de 78,4% et un taux d'enrobant de 11,7 % (exemple 3) et 640g de granulés ayant un titre en lysine, HCl de 47,1 % et un taux d'enrobant de 21,2 % (exemple 18).

### 4. Evaluation in vitro des caractéristiques de protection-libération des granulés pelliculés (exemple 3)

En milieu tampon à pH6, à 40°C, après 24 heures le taux de rétention de ces granulés est de 99,45%. A pH2 75% de la méthionine est libérée en 7 à 8 heures.

Les autres exemples sont réalisés selon le même mode opératoire mais avec soit des quantités de composés différentes soit des mélanges d'acides gras différents. Les résultats sont indiqués dans les tableaux 1 à 5.

### 5 Evaluation in sacco de la protection libération de la méthionine enrobée selon l'exemple 3

### 5.1 Procédure expérimentale

On utilise des sachets nylon de 6X6 cm ayant un diamètre de maille de 48 microns dans lesquels on introduit 1,5 g de produit conformément à l'exemple 3.Dans le rumen d'un vache canulée on introduit 1 sachet ruminal (20X20 cm, 100 microns de maille) contenant 15 sachets précédents. On laisse les sachets en place 24 heures dans le rumen. On lave les sachets à l'eau claire, on sèche 3 sachets à l'étuve 48 heures, on enregistre le poids.

Les 12 autres sachets sont immergés, 2,5 heures, avant réintroduction dans l'animal, dans une solution de pepsine à pH 2 (3 g de pepsine à 300 à 600 UI/ml/l). On introduit les autres sachets dans une vache par une canule duodénale lors du repas du soir. les sachets sont collectés dans les fécès le lendemain matin à 8 H. On les rince à l'eau, on sèche à l'étuve, on pèse.

### 5.2 Mesure de la méthionine résiduelle

A chaque étape on mesure la méthionine résiduelle. La méthionine disponible pour l'animal est déterminée par la différence entre la méthionine contenue après passage dans le rumen et la méthionine contenue dans les fécès divisée par la méthionine contenue dans le granulé initial. Les résultats des essais sont indiqués dans le tableau 6.

### 6. Evaluation in vivo des caractéristiques de protection-libération des granulés pelliculés

On utilise quatre vaches canulées du rumen et du duodénum. Elles sont alimentées par un mélange d'ensilage de maïs et de foin, une partie d'entre elles reçoivent une supplémentation en méthionine protégée de 50g de produit (préparé selon la composition de l'exemple 3) par animal distribué en 4 parts égales quatre fois par jour. On mesure la concentration plasmatique en méthionine qui est de 0.68 mg/100g comparée à celle d'un témoin ne recevant pas de méthionine qui est de 0.23mg/100g. Le flux de méthionine au niveau du duodénum est de 33.7 g/j au dessus du groupe témoin ne recevant pas de méthionine, ce flux correspond à une quantité ingérée de méthionine de 38.3g/j (50g de granulé contenant 76.6% de méthionine).

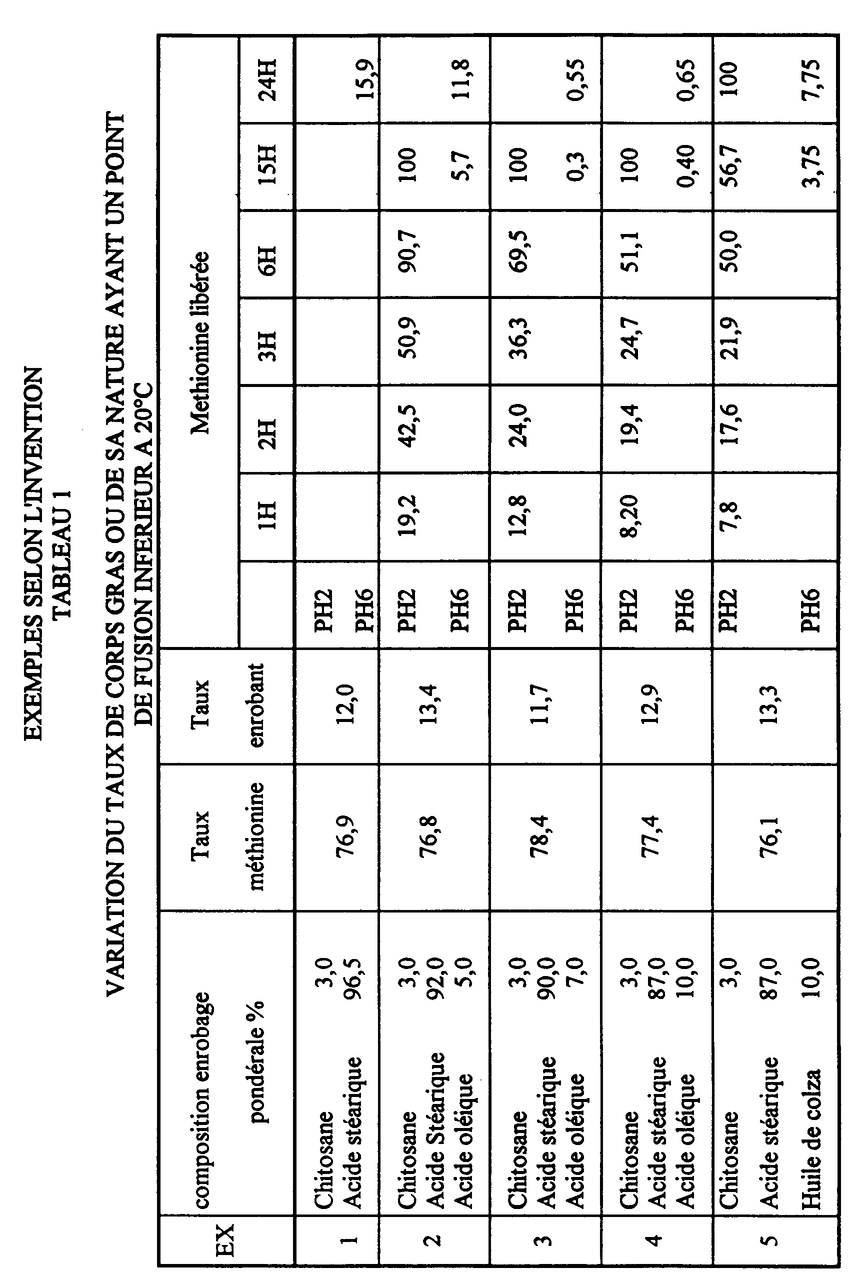

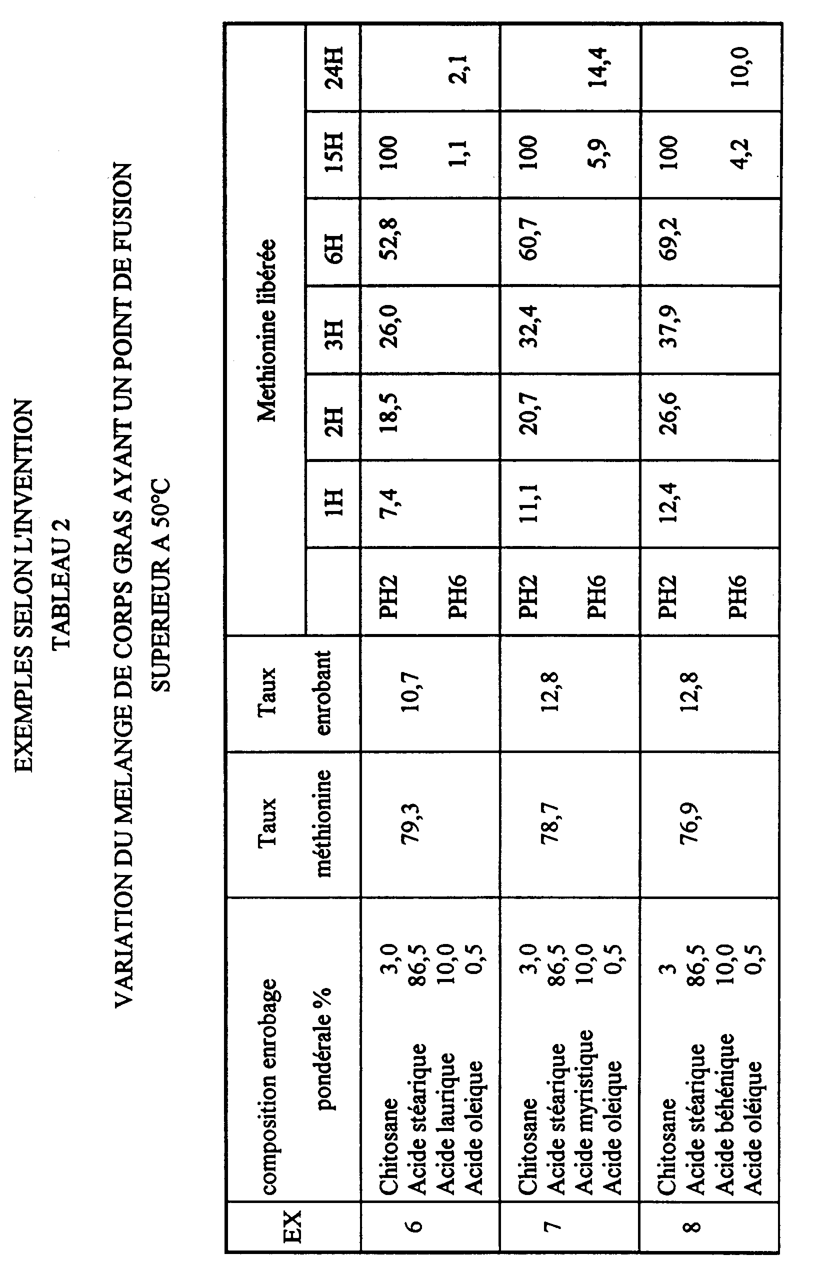

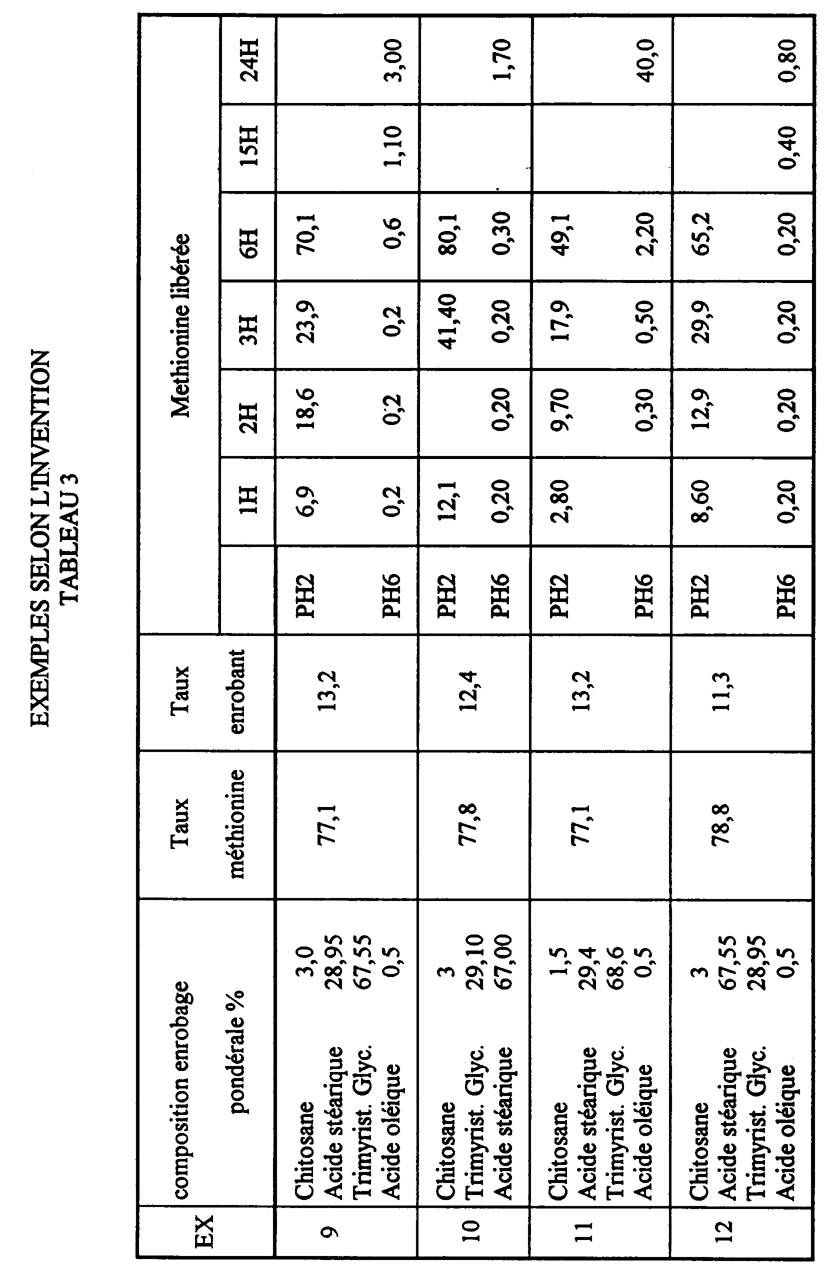

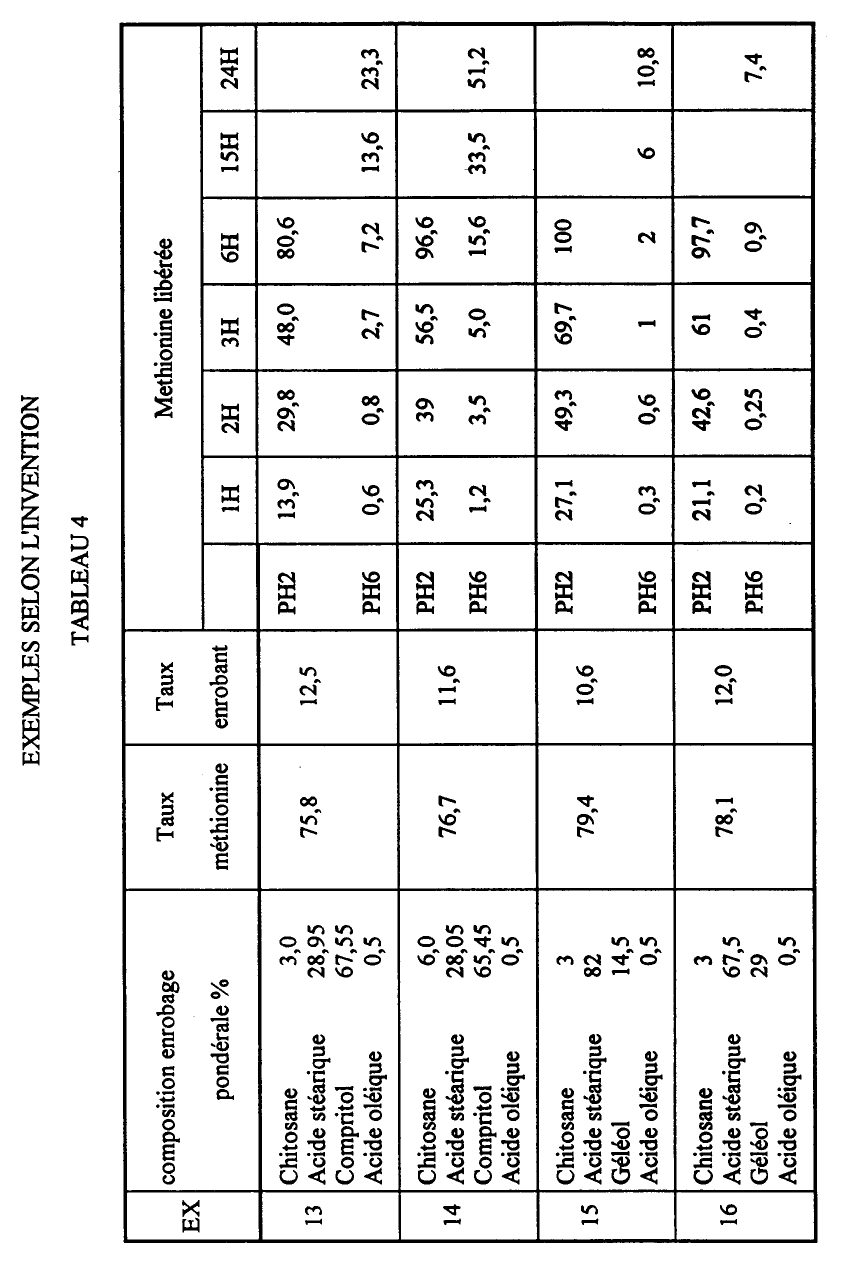

**TABLEAU 5**

| VARIATION DE LA SUBSTANCE BIOLOGIQUEMENT ACTIVE | | | | | | | | | | | |
|---|---|---|---|---|---|---|---|---|---|---|---|
| EX | Taux méthionine/Lysine HCl | Composition Enrobage | | Taux enrobant | Lysine HCl libérée | | | | | | |
| | | | | | | 1H | 2H | 3H | 6H | 15H | 24H |
| 17 | 13,3/47,1 | Chitosane | 2,0 | 21,7 | PH2 | 1 | 4,6 | 26,5 | 63,4 | 100 | |
| | | Acide stéarique | 68,0 | | | | | | | | |
| | | Acide oléique | 30,0 | | PH6 | | | | | 1 | 6,4 |
| 18 | 13,0/47,1 | Chitosane | 3,0 | 21,2 | PH2 | 7,3 | 25,4 | 43,9 | 80 | 100 | |
| | | Acide stéarique | 67,0 | | | | | | | | |
| | | Acide oléique | 30,0 | | PH6 | | | | | 0,8 | 8,7 |
| 19 | 13,2/47 | Chitosane | 5,0 | 21,4 | PH2 | 7,2 | 31,2 | 53,6 | 85,7 | 100 | |
| | | Acide stéarique | 65,0 | | | | | | | | |
| | | Acide oléique | 30,0 | | PH6 | | | | | 4,7 | 10,9 |

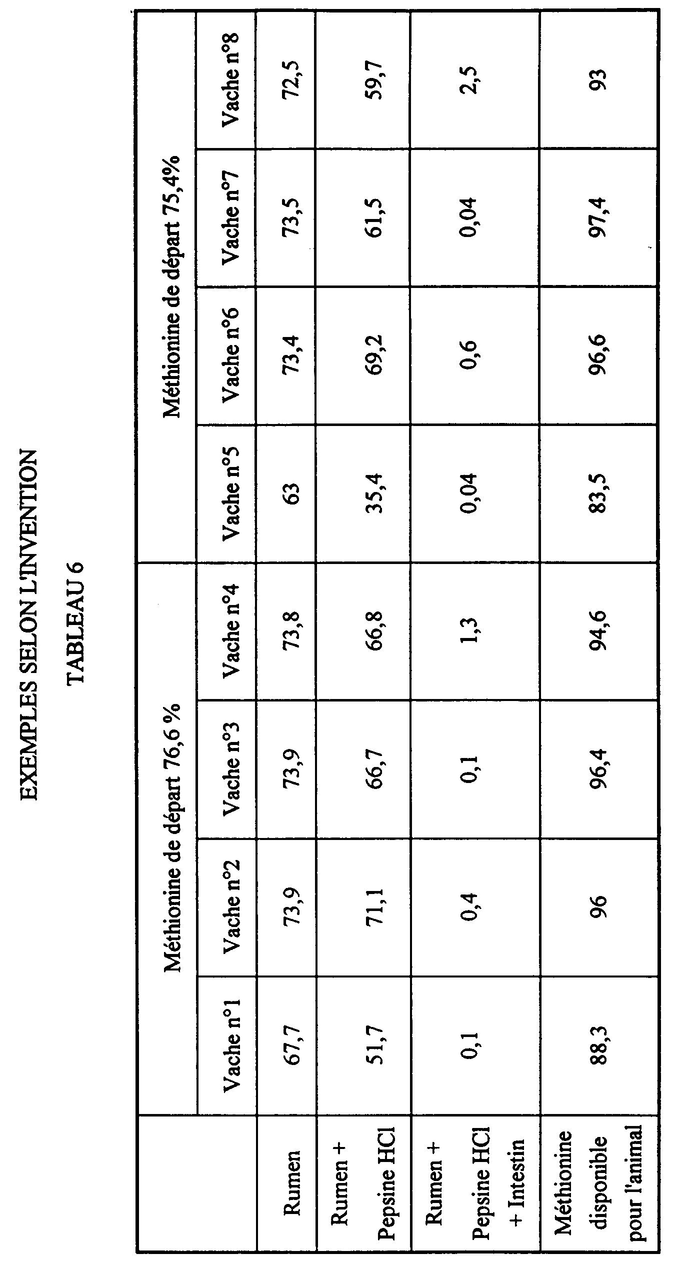

## Revendications

1. Compositions nutritives ou médicamenteuses pour l'administration aux ruminants contenant une ou plusieurs substances biologiquement actives sous forme granulaire enrobées par une composition d'enrobage contenant :
- 1 à 10 % de sel de chitosane exprimé en équivalent chitosane
- 99 à 90 % d'un corps gras ou d'un mélange de corps gras dont le point de fusion est supérieur à 45°C.

2. Compositions nutritives ou médicamenteuses selon la revendication 1 caractérisées en ce que la composition d'enrobage contient 1 à 5 % en poids de sels de chitosane.

3. Compositions nutritives ou médicamenteuses selon les revendications 1 et 2 caractérisées en ce que la composition d'enrobage contient
- 3 à 5 % de sel de chitosane
- 45 à 97 % d'un corps gras ou d'un mélange de corps gras ayant un point de fusion supérieur à 50°C
- 0 à 50 % d'un corps gras ou d'un mélange de corps gras ayant un point de fusion inférieur à 25°C.

4. Compositions nutritives ou médicamenteuses selon l'une quelconque des revendications 1 à 3 caractérisées en ce que la composition d'enrobage contient :
- 3 à 5 % de sel de de chitosane
- 55 à 92 % d'un corps gras ayant un point de fusion supérieur à 50°C
- 5 à 40 % d'un corps gras ou d'un mélange d'aides gras ayant un point de fusion inférieur à 25°C.

5. Compositions selon la revendication 1 caractérisées en ce que le principe actif est choisi parmi la méthionine et/ou la lysine.

6. Composition selon l'une quelconque des revendications 1 à 4 caractérisée en ce que le sel de chitosane est choisi parmi le chlorure, l'acétate, l'adipate, le citrate, le formiate, le glutamate, le lactate, le malonate, l'oxalate, le propionate, le pyruvate, le succinate, le tartrate.

7. Composition selon la revendication 6 caractérisé en que ce le sel de chitosane est l'acétate de chitosane.

8. Compositions selon la revendication 3 caractérisées en ce que le corps gras ou le mélange de corps gras ayant un point de fusion supérieur à 50°C est choisi parmi les acides gras, les esters d'acide gras, les alcools gras, les paraffines, les huiles végétales ou animales hydrogénées, les cires.

9. Compositions selon la revendication 8 caractérisées en ce que le corps gras ou le mélange de corps gras ayant un point de fusion supérieur à 50°C est choisi parmi l'acide stéarique, l'acide béhénique, l'acide laurique, l'acide myristique et la stéarine.

10. Compositions selon la revendication 8 caractérisées en ce que le corps gras ou le mélange de corps gras ayant un point de fusion supérieur à 50°C est choisi parmi les monoesters, diesters et triesters de glycérol et d'un acide gras.

11. Compositions selon la revendication 3 caractérisées en ce que le corps gras ou le mélange de corps gras ayant un point de fusion inférieur à 25°C est choisi parmi les acides gras insaturés ayant 12 à 22 atomes de carbone.

12. Compositions selon la revendication 11 caractérisée en ce que le corps gras ou le mélange de corps gras ayant un point de fùsion inférieur à 25°C est choisi parmi l'acide oléique, l'huile de colza, de tournesol, de coprah, d'arachide, de maïs et d'olive.

13. Compositions nutritives à base de méthionine selon l'une quelconque des revendications précédentes caractérisées en ce que la composition d'enrobage contient:
- 3 % d'acétate de chitosane
- 90 % d'acide stéarique
- 7 % d'acide oléique.

14. Compositions selon les revendications 1 et 5 caractérisées en ce que le granulé de principe actif a un diamètre compris entre 0,6 et 2,5 mm.

15. Compositions selon la revendication 1 caractérisées en ce que l'épaisseur de la couche d'enrobage est comprise entre 20 µm et 200 µm et de préférence entre 60 µm et 120 µm.

16. Compositions selon les revendications 14 et 15 caractérisées en ce qu'elles contiennent 60 à 90 % de principes actifs sous forme granulaire et 4 à 25 % en poids de composition d'enrobage selon l'une quelconque des revendications 1 à 13.

17. Procédé de préparation des compositions selon l'une quelconque des revendications 1 et 14 à 16 caractérisées en ce que l'on forme une émulsion aqueuse des constituants de la composition d'enrobage que l'on pulvérise sur les granulés de principes actifs.

18. Procédé de préparation des compositions selon la revendication 17 caractérisé en ce que dans une première étape on prépare une solution aqueuse de sel de chitosane à laquelle on ajoute le corps gras ayant un point de fusion inférieur à 25°C puis sous agitation on ajoute le corps gras ou le mélange de corps gras ayant un point de fusion supérieur à 50°C, puis dans une deuxième étape on pulvérise l'émulsion obtenue sur des granulés de principe actif.

## Claims

1. Nutrient or medicinal compositions for administration to ruminants containing one or a number of biologically active substances in the granular form coated with a coating composition containing:
- 1 to 10 % of chitosan salt, expressed as chitosan equivalent
- 99 to 90 % of a fatty substance or of a mixture of fatty substances whose melting point is greater than 45°C.

2. Nutrient or medicinal compositions according to claim 1, characterized in that the coating composition contains 1 to 5 % by weight of chitosan salts.

3. Nutrient or medicinal compositions according to claims 1 and 2, characterized in that the coating composition contains
- 3 to 5 % of chitosan salt
- 45 to 97 % of a fatty substance or of a mixture of fatty substances having a melting point greater than 50°C
- 0 to 50 % of a fatty substance or of a mixture of fatty substances having a melting point of less than 25°C.

4. Nutrient or medicinal compositions according to any one of claims 1 to 3, characterized in that the coating composition contains:
- 3 to 5 % of chitosan salt
- 55 to 92 % of a fatty substance having a melting point greater than 50°C
- 5 to 40 % of a fatty substance or of a mixture of fatty substances having a melting point of less than 25°C.

5. Compositions according to claim 1, characterized in that the active principle is chosen from methionine and/or lysine.

6. Composition according to any one of claims 1 to 4, characterized in that the chitosan salt is chosen from the chloride, the acetate, the adipate, the citrate, the formate, the glutamate, the lactate, the malonate, the oxalate, the propionate, the pyruvate, the succinate or the tartrate.

7. Composition according to claim 6, characterized in that the chitosan salt is chitosan acetate.

8. Compositions according to claim 3, characterized in that the fatty substance or the mixture of fatty substances having a melting point greater than 50°C is chosen from fatty acids, fatty acid esters, fatty alcohols, paraffins, hydrogenated vegetable or animal oils, or waxes.

9. Compositions according to claim 8, characterized in that the fatty substance or the mixture of fatty substances having a melting point greater than 50°C is chosen from stearic acid, behenic acid, lauric acid, myristic acid and stearin.

10. Compositions according to claim 8, characterized in that the fatty substance or the mixture of fatty substances having a melting point greater than 50°C is chosen from the monoesters, diesters and triesters of glycerol and of a fatty acid.

11. Compositions according to claim 3, characterized in that the fatty substance or the mixture of fatty substances having a melting point of less than 25°C is chosen from unsaturated fatty acids having 12 to 22 carbon atoms.

12. Compositions according to claim 11, characterized in that the fatty substance or the mixture of fatty substances having a melting point of less than 25°C is chosen from oleic acid or rapeseed, sunflower, coconut, groundnut, maize and olive oil.

13. Nutrient compositions based on methionine according to any one of the preceding claims, characterized in that the coating composition contains:
- 3 % of chitosan acetate
- 90 % of stearic acid
- 7 % of oleic acid.

14. Compositions according to claims 1 and 5, characterized in that the active principle granule has a diameter of between 0.6 and 2.5 mm.

15. Compositions according to claim 1, characterized in that the thickness of the coating layer is between 20 µm and 200 µm and preferably between 60 µm and 120 µm.

16. Compositions according to claims 14 and 15, characterized in that they contain 60 to 90 % of active principles in the granular form and 4 to 25 % by weight of coating composition according to any one of claims 1 to 13.

17. Process for the preparation of the compositions according to any one of claims 1 and 14 to 16, characterized in that an aqueous emulsion of the constituents of the coating composition is formed and is sprayed onto the active principle granules.

18. Process for the preparation of the compositions according to claim 17, characterized in that, in a first stage, an aqueous chitosan salt solution is prepared, to which the fatty substance having a melting point of less than 25°C is added, then the fatty substance or the mixture of fatty substances having a melting point greater than 50°C is added with stirring and then, in a second stage, the emulsion obtained is sprayed onto active principle granules.

## Patentansprüche

1. Nährstoff- oder Arzneimittel-Zusammensetzungen für die Verabreichung an Wiederkäuer, enthaltend eine oder mehrere biologisch aktive Substanzen in Form von Granulat und umhüllt durch eine Umhüllungszusammensetzung, die enthält:
- 1 bis 10 % Chitosansalz, ausgedrückt in Äquivalent Chitosan,
- 99 bis 90 % eines Fettkörpers oder einer Mischung von Fettkörpern, deren Schmelzpunkt über 45 °C liegt.

2. Nährstoff- oder Arzneimittel-Zusammensetzungen nach Anspruch 1, dadurch gekennzeichnet, daß die Umhüllungszusammensetzung 1 bis 5 Gew.-% Chitosansalze enthält.

3. Nährstoff- oder Arzneimittel-Zusammensetzungen nach Anspruch 1 und 2, dadurch gekennzeichnet, daß die Umhüllungszusammensetzung enthält:
- 3 bis 5 % Chitosansalz,
- 45 bis 97 % eines Fettkörpers oder einer Mischung von Fettkörpern, deren Schmelzpunkt über 50 °C liegt,
- 0 bis 50 % eines Fettkörpers oder einer Mischung von Fettkörpern, deren Schmelzpunkt unter 25 °C liegt.

4. Nährstoff- oder Arzneimittel-Zusammensetzungen nach irgendeinem der Ansprüche 1 bis 3, dadurch gekennzeichnet, daß die Umhüllungszusammensetzung enthält:
- 3 bis 5 % Chitosansalz,
- 55 bis 92 % eines Fettkörpers mit einem Schmelzpunkt von über 50 °C,
- 5 bis 40 % eines Fettkörpers oder einer Mischung von Fettkörpern mit einem Schmelzpunkt von unter 25 °C.

5. Zusammensetzungen nach Anspruch 1, dadurch gekennzeichnet, daß der Wirkstoff unter Methionin und/oder Lysin ausgewählt wird.

6. Zusammensetzungen nach irgendeinem der Ansprüche 1 bis 4, dadurch gekennzeichnet, daß das Chitosansalz unter dem Chlorid, Acetat, Adipat, Citrat, Formiat, Glutamat, Lactat, Malonat, Oxalat, Propionat, Pyruvat, Succinat, Tartrat ausgewählt wird.

7. Zusammensetzungen nach Anspruch 6, dadurch gekennzeichnet, daß das Chitosansalz das Chitosanacetat ist.

8. Zusammensetzungen nach Anspruch 3, dadurch gekennzeichnet, daß der Fettkörper oder die Mischung von Fettkörpern mit einem Schmelzpunkt von über 50 °C unter den Fettsäuren, den Fettsäureestern, den Fettalkoholen, den Paraffinen, den hydrierten pflanzlichen oder tierischen Ölen und den Wachsen ausgewählt werden.

9. Zusammensetzungen nach Anspruch 8, dadurch gekennzeichnet, daß der Fettkörper oder die Mischung von Fettkörpern mit einem Schmelzpunkt von über 50 °C unter Stearinsäure, Behensäure, Laurinsäure, Myristinsäure und Stearin ausgewählt werden.

10. Zusammensetzungen nach Anspruch 8, dadurch gekennzeichnet, daß der Fettkörper oder die Mischung von Fettkörpern mit einem Schmelzpunkt von über 50 °C unter den Monoestern, Diestern oder Triestern von Glycerin und einer Fettsäure ausgewählt werden.

11. Zusammensetzungen nach Anspruch 3, dadurch gekennzeichnet, daß der Fettkörper oder die Mischung von Fettkörpern mit einem Schmelzpunkt von unter 25 °C unter den ungesättigten Fettsäuren mit 12 bis 22 Kohlenstoffatomen ausgewählt werden.

12. Zusammensetzungen nach Anspruch 11, dadurch gekennzeichnet, daß der Fettkörper oder die Mischung von Fettkörpern mit einem Schmelzpunkt von unter 25 °C unter Oleinsäure, Colzaöl, Sonnenblumenöl, Copraöl, Erdnußöl, Maisöl und Olivenöl ausgewählt werden.

13. Nährstoff-Zusammensetzungen auf der Basis von Methionin nach irgendeinem der vorstehenden Ansprüche, dadurch gekennzeichnet, daß die Umhüllungszusammensetzung enthält:
- 3 % Chitosanacetat,
- 90 % Stearinsäure,
- 7 % Oleinsäure.

14. Zusammensetzungen nach Anspruch 1 und 5, dadurch gekennzeichnet, daß das Granulat des Wirkstoffes einen Durchmesser zwischen 0,6 mm und 2,5 mm besitzt.

15. Zusammensetzungen nach Anspruch 1, dadurch gekennzeichnet, daß die Dicke der Umhüllungsschicht zwischen 20 µm und 200 µm und vorzugsweise zwischen 60 µm und 120 µm beträgt.

16. Zusammensetzungen nach Anspruch 14 und 15, dadurch gekennzeichnet, daß sie 60 % bis 90 % Wirkstoff in Form von Granulat und 4 Gew.-% bis 25 Gew.-% Umhüllungszusammensetzung nach einem der Ansprüche 1 bis 13 enthalten.

17. Verfahren zur Herstellung der Zusammensetzungen nach irgendeinem der Ansprüche 1 und 14 bis 16, dadurch gekennzeichnet, daß man eine wäßrige Emulsion der Bestandteile der Umhüllungszusammensetzung bildet, die man auf die Granulate des Wirkstoffes sprüht.

18. Verfahren zur Herstellung der Zusammensetzungen nach Anspruch 17, dadurch gekennzeichnet, daß man in einer ersten Stufe eine wäßrige Lösung des Chitosansalzes herstellt, zu der man den Fettkörper mit einem Schmelzpunkt von unter 25 °C gibt, und man anschließend unter Rühren den Fettkörper oder die Mischung von Fettkörpern mit einem Schmelzpunkt von über 50 °C hinzugibt, wonach man in einer zweiten Stufe die erhaltene Emulsion auf die Granulate des Wirkstoffes sprüht.
